# EUROPEAN PATENT APPLICATION

(11) **EP 4 205 849 A1**
(43) Date of publication of application: **05.07.2023**
(21) Application number: 21218385.9
(22) Date of filing: 30.12.2021
(51) Int. Cl.: B01L 3/00, B01L 7/04, B01L 1/02, B01L 7/00, C12M 1/34

(54) **SYSTEM AND METHOD FOR THERMAL TREATMENT OF CANCER CELLS**

(71) Applicant: Elmedix NV, 3001 Leuven (BE)
(72) Inventor: Van den Bossche, Johan, 3210 Linden (BE); Bogers, John-Paul, 2530 Boechout (BE); Brancato, Luigi, 3010 Leuven (BE)
(74) Representative: IPLodge bv

(57) **Abstract**

The invention pertains to a system for controlling the temperature of at least one biological sample, the system comprising: at least one thermally insulated treatment chamber suitable for each containing one or more of said at least one biological sample, each in its individual recipient, wherein each one of said at least one thermally insulated treatment chamber further comprises: a thermal means; one or more first biocompatible temperature sensors, wherein each of said biocompatible temperature sensors is insertable in said corresponding sample; one or more second temperature sensors for providing a signal indicative of the air temperature in said thermally insulated treatment chamber; and at least one controller configured for steering thermal means by taking into account at least a signal of said one or more first biocompatible temperature sensors and of said one or more second temperature sensors. The invention further pertains to a method for use in same.

## Description

### Technical Field

The present invention relates to a system for controlling the temperature of at least one biological sample and to observe the effect of a thermal treatment on said at least one biological sample, said biological sample being contained by at least one thermally insulated treatment chamber of said system. The invention further pertains to a method for use in same and to a method for defining a thermal treatment or thermal treatment strategy for treating a biological sample of cancerous cells.

### Background Art

Thermal treatment is a known cancer therapy which involves increasing the overall body temperature of a patient for a period of time, thereby inducing a state of hyperthermia in the body. It is known that tumour cells are sensitive to stress conditions, such as elevated temperatures. Indeed, temperatures between 40 °C and 44 °C are cytotoxic for cells in an environment with a low pO₂ and low pH, conditions which are found within tumour tissue, due to insufficient blood perfusion. Thermal treatment systems for temporarily increasing the body temperature of a patient have been described in literature. WO 2019/211411, filed by the inventor, describes a system for inducing a state of hyperthermia in the body of a patient.

Although a patient can thus be accurately monitored and safely subjected to a state of hyperthermia, the thermal treatment systems as known in the state of the art do not provide any information as to which treatment protocol delivers the best results for the patient. Currently, the planning of thermal treatment sessions is mainly done through computer simulations. These simulations necessarily make assumptions about the response of the cancerous tissue to a condition of hyperthermia. Considering that a complete thermal treatment therapy of a cancer patient may include several distinct sessions, it is of utmost importance to know the effect of parameters such as session frequency, maximum temperature obtained during a session and/or temperature profile during a session, session duration and length of a complete therapy, on a tumour. If the response of cancerous tissue to such parameters were known through measurements, more accurate treatment planning would be possible tailored for the patient, possibly resulting in a smaller amount of treatment sessions, reduced cost, risk and discomfort and improved outcome.

Cancerous tissue can be studied in vitro or *in ovo* (Eckrich, J. et al. "Monitoring of tumor growth and vascularization with repetitive ultrasonography in the chicken chorioallantoic-membrane-assay", Sci Rep 10, 18585 (2020), https://doi.org/10.1038/s41598-020-75660-y ). For studying the effect of a thermal treatment, samples of tumour cells can be studied in an incubator, which refers herein to a chamber used to provide controlled environmental conditions for the cultivation or study of microorganisms. However, the inventors have found that incubators known in the state of the art do not permit to achieve the goals set herein, due to the fact that sample temperature reporting and controlling in traditional incubators is not accurate enough. On the contrary, it has been found that in order to observe the effect of a thermal treatment on a cancerous cell culture, temperature control of the chamber environment and more in particular of the cell culture or biological sample is of crucial importance.

There therefore remains a need for a tool and method that allows observing and evaluating the effectiveness of a thermal treatment or thermal treatment strategy in treating tumour cells, wherein treating tumour cells relates to the treatment's ability to destroy a maximum amount of said tumour or cancer cells. Furthermore, there is a need for a system that allows to subject biological samples, containing tumour cells, to such a strict thermal treatment protocol wherein the temperature of the sample is controlled, measured and steered with high accuracy and wherein a target temperature imposed by a thermal treatment protocol is obtained within the shortest delay, without overshooting or undershooting said temperature. It is furthermore important that such a system allows investigating the effect of thermal treatment in combination with other therapies, such as chemotherapy and/or irradiation therapy.

### Summary of the invention

According to an aspect of the present invention, there is provided a system for controlling the temperature of at least one biological sample in at least one thermally insulated chamber;
wherein said system comprises:
- at least one thermally insulated treatment chamber suitable for each containing one or more of said at least one biological sample, each in its individual recipient;
   wherein each one of said at least one thermally insulated treatment chamber further comprises:
   o a thermal means for altering the temperature of said one or more of said at least one biological sample, when placed inside said thermally insulated treatment chamber;
   o one or more first biocompatible temperature sensors, wherein each of said one or more first biocompatible temperature sensors provides a first sensor signal indicative of the temperature in a corresponding sample of said one or more of said at least one biological sample, wherein each of said biocompatible temperature sensors is insertable in said corresponding sample;
   o one or more second temperature sensors, wherein each of said one or more second temperature sensors provides a second sensor signal indicative of the air temperature in said thermally insulated treatment chamber;
- at least one controller configured for steering thermal means of a corresponding treatment chamber of said at least one treatment chamber by taking into account at least said one or more first sensor signal and said one or more second sensor signal of said corresponding treatment chamber.

It is an advantage of the present invention that the system as described herein allows to subject at least one biological sample to a thermal protocol, which protocol may be defined in terms of temporarily and/or alternately increasing and/or decreasing the temperature (heating or cooling) of a biological sample placed in a thermally insulated chamber for a certain duration or period of time. Advantageously, the system can contain more than one treatment chamber, allowing samples, e.g. cell cultures with tumour cells of an individual patient, to undergo different thermal protocols simultaneously, allowing to compare protocols for their therapeutic effectiveness within a short span of time. Advantageously, the system can contain more than one sample per treatment chamber, which more than one sample is by consequence subjected to the same thermal protocol at the same time. Such a design allows for samples, e.g. cell cultures with tumour cells of an individual patient, to be treated with a variety of e.g. chemotherapeutic compounds, which compounds can subsequently be compared for their therapeutic effectiveness. Furthermore, by having a plurality of biological samples subjected to a same treatment in a treatment chamber, biological variability between different samples can be eliminated.

It is a further advantage of the present invention that the system allows for observation of the effects of one or more different thermal treatments on biological samples. Based on the reaction of said samples to a variety of thermal treatments, possibly in combination with other therapies, a thermal treatment protocol may be selected for use in a patient.

Advantageously, the system allows subjecting said at least one biological sample to a thermal therapy with high temperature accuracy, wherein the temperature course of said at least one biological sample can be controlled, measured and/or steered with high accuracy.

Advantageously, the use of biocompatible sensors allows to measure the temperature inside the biological sample, without interfering with or influencing any biological or metabolic activities inside said sample. Indeed, by simultaneously measuring the air temperature inside a treatment chamber and the temperature of a sample inside said chamber, the air temperature may be adjusted to compensate for metabolic heat production within the biological sample, ensuring that the exact desired treatment temperature is reached within the biological sample. As such, deviations from a thermal protocol can be kept to an acceptable minimum. In other words, by measuring both the air temperature inside a treatment chamber and the temperature inside the biological sample, temperature overshooting and/or undershooting, and therefore protocol uncertainty, can be avoided or kept to an acceptable minimum.

It is thereby to be noted that said metabolic activity can change during the protocol, as an effect of changes in temperature, or in reaction to e.g. administration of drugs, such as chemotherapeutic compounds. In the case of chicken-egg embryos, metabolic heat production even changes significantly depending on the embryo development stage. It is an advantage of the system that it adapts continuously to such slow and fast changes ensuring that the cancer cells within the biological sample are kept at the desired temperature.

It is a further advantage of the system that said thermal insulation prevents loss of heat via the walls of the treatment chamber, further increasing the accuracy of temperature control of the sample.

It is a further advantage that an individual recipient allows individual handling of said biological samples to, from and inside said treatment chamber. Individual handling may refer to inserting, removing from and organizing samples inside said treatment chamber.

In an embodiment of the system according to the present invention, the controller is configured to steer said thermal means on the basis of a thermal protocol, which is typically pre-programmed.

In an embodiment of the system according to the present invention, said at least one controller relates to one single controller for steering the thermal means of each of said at least one thermally insulated treatment chamber by taking into account the corresponding said one or more first sensor signal and said one or more second sensor signal.

It is an advantage of said embodiment that only one controller is needed to process data from, control and steer one or more thermally insulated chambers and the samples they contain. Hence, the system can be made both cheaper and more compact.

In an embodiment of the system according to the present invention, each of said at least one thermally insulated treatment chamber contains one biological sample.

It is an advantage of said embodiment that the controller, by interpretation of said one or more first sensor signal and said one or more second sensor signal, only has to take into account the metabolic activity and possible production of heat of only one biological sample, which facilitates the overall process of steering and controlling of the sample temperature.

In an embodiment of the system according to the present invention, said thermal means comprise at least one of the following:
- a device for heating and/or cooling fluid, propellers, and related conducts for forcedly circulating said fluid through a corresponding treatment chamber;
- a resistive heating element for heating the air in a corresponding thermally insulated treatment chamber;
- a cooling element for cooling the air in a corresponding thermally insulated treatment chamber;
- a radiation heating element.

It is an advantage of said embodiment that each thermally insulated chamber disposes of its own thermal means which are dedicated to increase or decrease the temperature in the air of the chamber. It has been found that forced circulation of a fluid in conducts within the chamber allows for faster and more uniform control of any biological samples contained in the chamber. It has further been found that the use of resistive heating elements, radiation heating elements and cooling elements further provide for a fast heating or fast cooling respectively of the environment in the chamber.

In an embodiment of the system according to the present invention, said thermal means comprise a fluid, a device for heating and/or cooling said fluid and related conducts for forcedly circulating said fluid through or within a corresponding treatment chamber, and a compartment for temperature conditioning of said fluid.

It is an advantage of said embodiment that the use of a compartment for temperature conditioning of a fluid to be circulated inside the treatment chamber, preferably via a closed loop, further increases the rapidity with which the system can react to any temperature changes measured in the biological sample, due to e.g. metabolic processes in the sample.

In an embodiment of the system according to the present invention, each one of said at least one thermally insulated treatment chamber further comprises at least one sensor to monitor one or more of relative humidity, oxygen and carbon dioxide levels inside said treatment chamber.

It is an advantage of said embodiment that environmental conditions having a negative effect on the samples may be detected in time.

In an embodiment of the system according to the present invention, each one of said at least one thermally insulated treatment chamber comprises means to regulate one or more of humidity, oxygen and carbon dioxide levels inside said treatment chamber.

It is an advantage of said embodiment that environmental conditions having a negative effect on the samples may be countered in time.

In an embodiment of the system according to the present invention, at least one of said at least one treatment chamber is further provided with at least one weighing scale for continuously evaluating the weight of said at least one biological sample.

In an embodiment of the system according to the present invention, at least one of said at least one thermally insulated treatment chamber is further provided with at least one camera for providing visual inspection of said one or more of said at least one biological sample, and preferably at least one light source for illuminating said one or more of said at least one biological sample.

It is an advantage of said embodiment that cell parameters, such as tumour size in function of time, can be monitored, recorded and evaluated during the treatment without perturbing the temperature.

In an embodiment of the system according to the present invention, at least one of said at least one thermally insulated treatment chamber is further provided with an optical detection system with fluorescence for inspection and continuous monitoring of said one or more of said at least one biological sample.

It is an advantage of said embodiment that cell parameters, such as cell activity or tumour size in function of time, can be monitored, recorded and evaluated during the treatment without perturbing the temperature.

In an embodiment of the system according to the present invention, at least one of said at least one thermally insulated treatment chamber is further provided with an accelerometer.

In an embodiment of the system according to the present invention, said at least one biological sample refers to one of the following: a cell culture, an egg, preferably a fertilized egg.

In an embodiment of the activator composition according to the present invention, said at least one biological sample contains tumour cells.

In an embodiment of the system according to the present invention, said at least one controller comprises a PID controller.

It is an advantage of said embodiment that overshooting of a desired temperature or target temperature can be avoided.

According to an aspect of the present invention, there is provided a use of the system as described herein for defining, determining or selecting a thermal treatment for treating a biological sample of tumour cells.

It is advantage of said use that a thermal treatment, protocol or thermal treatment strategy may be selected for treating a culture of tumours cells, resulting in a maximal tumour cell death. Advantageously, such thermal treatment, protocol or strategy may also be selected for treating a patient's body.

In embodiments of said use according to the present invention, said defining, determining or selecting a thermal treatment includes evaluation by at least one selected from the following: visual monitoring with camera, monitoring with fluorescence, monitoring with an accelerometer, continuously weighing the sample.

According to an aspect of the present invention, there is provided a method for controlling the temperature of at least one biological sample in a thermally insulated treatment chamber, the method comprising the following steps:
- placing one or more of said at least one biological sample in said thermally insulated treatment chamber;
- inserting one or more first biocompatible temperature sensors in said one or more of said at least one biological sample, each of said one or more first biocompatible temperature sensors providing a first sensor signal indicative of the temperature in a corresponding sample of said one or more of said at least one biological sample; and
- steering thermal means of said thermally insulated treatment chamber for altering the temperature of said one or more of said at least one biological sample by use of a controller by taking into account at least said first sensor signal and a second sensor signal of said corresponding treatment chamber, provided by one or more second temperature sensors, each providing a second sensor signal indicative of the air temperature in said thermally insulated treatment chamber.

In an embodiment of the method according to the present invention, steering said thermal means relates to steering said thermal means for altering the temperature of said one or more of said at least one biological sample on the basis of a thermal protocol.

In an embodiment of the method according to the present invention, said method further comprises the following step:
- evaluating and/or comparing an effect or reaction of said at least one biological sample to said temperature alterations, and
- selecting a thermal treatment for treating said biological sample, on the basis of said effect to said temperature alterations.

In an embodiment of the method according to the present invention, evaluating and/or comparing an effect or reaction includes evaluation by at least one selected from the following: visual monitoring with camera, monitoring with fluorescence, monitoring with an accelerometer, continuously weighing the sample.

According to an aspect of the present invention, there is provided a method for defining and/or selecting a thermal treatment for treating a culture of tumour cells, the method comprising the following steps:
- placing one or more of at least one biological sample of said culture of tumour cells in a thermally insulated treatment chamber;
- inserting one or more first biocompatible temperature sensors in said one or more of said at least one biological sample, each of said one or more first biocompatible temperature sensors providing a first sensor signal indicative of the temperature in a corresponding sample of said one or more of said at least one biological sample;
- steering thermal means of said thermally insulated treatment chamber for altering the temperature of said one or more of said at least one biological sample by use of a controller by taking into account at least said first sensor signal and a second sensor signal of said corresponding treatment chamber, provided by one or more second temperature sensors, each providing a second sensor signal indicative of the air temperature in said thermally insulated treatment chamber,
- evaluating and/or comparing an effect or reaction of said at least one biological sample to said temperature alterations, and
- selecting a thermal treatment for treating said culture of tumour cells, on the basis of said effect to said temperature alterations.

In an embodiment of the method according to the present invention, steering said thermal means relates to steering said thermal means for altering the temperature of said one or more of said at least one biological sample on the basis of a thermal protocol.

In an embodiment of the method according to the present invention, evaluating and/or comparing an effect or reaction includes evaluation by at least one selected from the following: visual monitoring with camera, monitoring with fluorescence, monitoring with an accelerometer, continuously weighing the sample.

### Brief Description of the Drawings

Various technical effects and advantages of embodiments of the present invention will now be described with reference to the accompanying figures, in which:
- Figure 1 is an exemplary illustration of an embodiment of the system in accordance with the present invention;
- Figure 2 is an exemplary illustration of another embodiment of the system in accordance with the present invention.

### Detailed description of the invention

A specific application of the system **10,100** and methods as described herein relates to subjecting biological samples to a thermal protocol. More in particular, a specific application is to subject biological samples, derived or obtained from a patient, said samples containing cancerous cells or tumour cells from the patient, to a thermal treatment which treatment can also be administered to the body of the patient. Thermal treatment of a cancer patient in combination with other therapies such as irradiation and chemotherapy typically comprises a plurality of distinct thermal treatment sessions, during which the patient's body temperature is temporarily increased. Such a session is thereby characterized by a specific protocol, which protocol may differ from session to session.

It has surprisingly been found that the observed impact in terms of tumour cell death of a specific thermal treatment on a cell culture of tumour cells of an individual patient provides at least partly an indication of the effectiveness of such specific thermal treatment on the body of said individual patient. The skilled person will however appreciate that the immune reaction of an individual patient to said specific thermal treatment is not taken into account.

A thermal protocol relates herein to a plan defined by a set of parameters, expressing a sequence of distinct temperatures to which the biological sample, or in a possible later stage the patient's body, is subjected for a certain duration. Typically, the temperature of a biological sample in a thermally insulated chamber will be temporarily and/or alternately increased or decreased for a certain duration or period of time. It will be understood herein that such temperature alterations are typically steered by the controller on the basis of a defined, pre-programmed, thermal protocol.

Thermal protocol parameters may include
- the duration or length of time of a treatment session.
- the heating and cooling rates (speed of heating, typically expressed in °C/min);
- the temperature trend or temperature profile of a session.
- the maximum temperature attained for the body during a session.
- session frequency.
- the total length of the thermal treatment therapy, in terms of total number of sessions and time in between sessions.

Hence, a controller will typically steer the temperature of biological samples in said treatment chambers on the basis of predefined temperatures, temperature profiles and durations, making up the protocol.

In embodiments according to the invention, the temperature of said biological sample will be increased during the execution of a thermal protocol at least once to a minimum temperature of at least 37°C, preferably at least 38°C, more preferably at least 39°C, even more preferably at least 40°C, and most preferably at least 41°C. It will be understood that the temperature of said biological sample will be increased during the execution of a thermal protocol to a maximum temperature of at most 46°C, preferably at most 45°C, more preferably at most 44°C, and most preferably at most 43°C.

Typically, the temperature of said biological sample will be increased to a temperature in the range of 40.5°C to 43°C, preferably in the range of 41°C to 42.5°C, more preferably in the range of 41.3°C to 41.7°C, during the execution of a thermal protocol. More in particular, during the thermal treatment, the temperature of the sample will be raised at least once to the aforementioned temperature ranges.

The thermal means as described herein are configured to raise the temperature of the biological sample to a temperature that may be at least 37°C, 37.5°C, 38°C, 38.5°C, 39°C, 39.5°C, 40°C, 40.5°C, 41°C, 41.5°C, 42°C, 42.5°C or 43°C, typically 41.5°C +/- 0.1°C or 41.5°C.

In embodiments according to the invention, the at least one biological sample **20a, 20b, 20c, 20d, 200a, 200b** refers to one of the following: a cell culture, such a cell culture containing tumour cells, an egg, preferably a fertilized egg or egg with an embryo, wherein said egg, fertilized egg or egg with an embryo is provided with a cell culture of tumour cells. Typically, said egg will be a chicken egg.

It is known to use eggs of birds, typically a chicken egg, for *in ovo* tumour research. Typically, on day 3 or 4 of incubation, cancer cells or tumour cells are transplanted on top of the chorioallantoic membrane that surrounds a chicken embryo, allowing a tumour to grow within a short period of time, typically a few days.

According to the invention, the system **10,100** for controlling the temperature of at least one biological sample **20a, 20b, 20c, 20d, 200a, 200b,** as well as the methods as described herein, comprise at least one thermally insulated treatment chamber **30a, 30b, 300a, 300b** or incubator suitable for each containing one or more of said at least one biological sample **20a, 20b, 20c, 20d, 200a, 200b,** each in its individual recipient **21a, 21b, 21c, 21d, 210a, 210b.**

In embodiments according to the invention, the system **10,100** contains one, two, three, four, five, six, seven, eight, nine, ten or more thermally insulated treatment chambers **30a, 30b, 300a, 300b.**

As used herein, the term "thermally insulated treatment chamber" refers to a confined space in which one or more biological samples may be inserted or arranged for the purpose of undergoing or being subjected to a thermal treatment as described herein.

Said thermally insulated treatment chamber **30a, 30b, 300a, 300b** may be a cabinet-like space, a chest, a box, a chamber, or the like. The thermally insulated treatment chamber **30a, 30b, 300a, 300b** may have the geometrical shape of a box (rectangular cuboid), or the geometrical shape of a cylinder. The thermally insulated treatment chamber **30a, 30b, 300a, 300b** is an insulated enclosure forming a fully enclosed space for holding said one or more biological sample during a thermal treatment or a thermal treatment session. 'Fully enclosed' shall be understood as forming a closed shell around the samples during the procedure. It will however be understood that such a shell may be pierced by e.g. tubes, conducts, cables or channelling means, e.g. for providing heating or cooling fluids for heating or cooling the content inside the chamber, more in particular the air inside the chamber and the biological samples contained therein. In embodiments, such conducts may relate to conducts for providing moisture or humidity to the interior environment of the treatment chamber **30a, 30b, 300a, 300b,** allowing to regulate relative humidity inside said treatment chamber **30a, 30b, 300a, 300b.** In embodiments, such conducts may relate to conducts for providing oxygen and/or carbon dioxide to the interior of the treatment chamber **30a, 30b, 300a, 300b.** Further to said tubes, conducts, cables or channelling means, the treatment chamber **30a, 30b, 300a, 300b** may be provided with an opening for inserting and removing said at least one biological sample. Typically, such an opening will be provided in a sidewall of the treatment chamber.

Thermally insulated' or 'heat-insulating' shall be understood as meaning minimizing (or reducing as much as practically achievable and/or to a level that does not significantly affect the internal heat balance) thermal exchange with the exterior environment by a convection and/or conduction and/or radiation. Thus, heat losses and heat fluxes from the biological sample to its surroundings (or vice versa), and/or from the treatment chamber to its surroundings (or vice versa) may be reduced to a low level, e.g. a minimum, ideally to zero.

For the purpose of insulating the treatment chamber, said chamber **30a, 30b, 300a, 300b** may be equipped with heat-reflecting materials on the interior walls. Preferably glass fibre is used although other materials known in the state of the art as suitable heat insulators may be used.

In embodiments according to the invention, the thermally insulated treatment chamber **30a, 30b, 300a, 300b** may be provided with at least one ventilator and/or propeller in the interior of the chamber. It has been found that the use of a ventilator and/or a propeller increases temperature uniformity of the air inside the chamber.

In embodiments according to the invention, the treatment chamber **30a, 30b, 300a, 300b** is at least partially made of an optically transparent material or materials.

In embodiments according to the invention, each of said at least one thermally insulated treatment chamber **30a, 30b, 300a, 300b** contains a single biological sample. It has been found that heat production in a biological sample having a very active, unknown or unpredictable metabolic rate can be adequately controlled when placed alone in a treatment chamber.

In alternative embodiments according to the invention, said at least one thermally insulated treatment chamber **30a, 30b, 300a, 300b** may contain two, three, four, five, six, seven, eight, nine, ten or more biological samples **20a, 20b, 20c, 20d, 200a, 200b.**

In preferred embodiments according to the invention, said at least one thermally insulated treatment chamber **30a, 30b, 300a, 300b** may contain at least twenty, thirty, forty, fifty, sixty, seventy, eighty, ninety, one hundred or more biological samples **20a, 20b, 20c, 20d, 200a, 200b.**

Each biological sample may be located in an individual recipient **21a, 21b, 21c, 21d, 210a, 210b,** wherein one single individual recipient is assigned to one single biological sample. Advantageously, this allows to manipulate, insert, remove, arrange, each sample, typically an egg, individually.

Preferably, a plurality of said individual recipients **21a, 21b, 21c, 21d, 210a, 210b** may form one object such as a container, microplate or microwell plate. Typically, a microplate is used wherein up to 96 biological samples **20a, 20b, 20c, 20d, 200a, 200b,** preferably cell cultures, may be located in 96 wells, representing individual recipients **21a, 21b, 21c, 21d, 210a, 210b,** of the microplate.

Advantageously, by using a sample size in the order of the number of wells in a microplate, biological variability between different samples can be eliminated.

According to embodiments, such recipients, microwell plates or containers are made of biologically inert materials, meaning that there is no interference between the metabolic processes in the sample and the recipient material.

According to the invention, said at least one thermally insulated treatment chamber **30a, 30b, 300a, 300b** further comprises a thermal means **31a, 31b, 310a, 310b** for altering the temperature of said one or more of said at least one biological sample **20a, 20b, 20c, 20d, 200a, 200b,** when placed inside said thermally insulated treatment chamber **30a, 30b, 300a, 300b.**

More in particular, said thermal means **31a, 31b, 310a, 310b** is configured for temporarily and/or alternately heating and/or cooling the treatment chamber **30a, 30b, 300a, 300b,** the chamber air and any biological samples contained therein. It will be understood that cooling of the chamber environment and the samples may occur at the end of a thermal treatment session or e.g. when a risk is perceived by the controller **40a, 40b, 400** that the sample temperature is bound to overshoot - or to deviate from - a sample target temperature as defined by the protocol. This may be the case when metabolic activity in the biological sample causes the sample to heat. By cooling the treatment chamber **30a, 30b, 300a, 300b,** the heat produced in the sample can be dissipated, such that the temperature in the sample is or is acceptably close to a preferred target temperature.

It has been found that without any adjusting, the difference between sample temperature on the one hand and chamber air temperature or target temperature of a thermal protocol on the other hand, can reach up to 1.0°C, due to metabolic activity in the sample. However, by taking the sample temperature and chamber air temperature into account in steering said thermal means, it has been found that deviations with respect to a target temperature imposed by a thermal treatment protocol can be kept to an acceptable minimum, typically 0.1°C or less, even 0.05°C or less, or even 0.01°C or less.

In embodiments according to the invention, said thermal means **31a, 31b, 310a, 310b** includes at least one of the following:
- a fluid (not shown in the figures), a device (not shown in the figures) for heating and/or cooling said fluid, and related conducts (not shown in the figures) for circulating said fluid through a corresponding treatment chamber **30a, 30b, 300a, 300b;**
- a resistive heating element (not shown in the figures) for heating the air in a corresponding thermally insulated treatment chamber **30a, 30b, 300a, 300b;**
- a cooling element (not shown in the figures) for cooling the air in a corresponding thermally insulated treatment chamber;
- a radiation heating element (not shown in the figures).

Hence, the thermal means **31a, 31b, 310a, 310b** may be configured to forcedly circulate heated or cooled fluid in a closed loop, wherein said closed loop passes at least partly through the interior of the treatment chamber **30a, 30b, 300a, 300b** of which the thermal means forms part via an inlet and an outlet.

The fluid may be a liquid, a gas, a gaseous mixture, or a mixture of gas and vapor, air, dry air or moist air. In embodiments according to the invention, said fluid is injected in the interior space of the treatment chamber **30a, 30b, 300a, 300b** via an inlet and leaves said space via an outlet.

In alternative embodiments, said fluid circulates through the interior space of the treatment chamber **30a, 30b, 300a, 300b** in channelling means, such that the fluid remains physically separated from the air in the treatment chamber.

In embodiments, said cooling element can be one of the following: a Peltier module or cooler, or an airconditioning element.

Typically, cooling elements, resistive heating elements and radiation heating elements, such as infrared heating elements, will be installed inside the treatment chamber, usually to the interior wall of the treatment chamber.

In embodiments, thermal means, particularly cooling elements, resistive heating elements and radiation heating elements, are not shared between two or more treatment chambers.

In preferred embodiments according to the invention, said thermal means **31a, 31b, 310a, 310b** include at least a fluid, a device for heating and/or cooling said fluid and related conducts, tubing or channelling means for circulating said fluid through a corresponding treatment chamber **30a, 30b, 300a, 300b,** and a compartment for temperature conditioning of said fluid.

In embodiments, said compartment for temperature conditioning is not shared between two or more treatment chambers **30a, 30b, 300a, 300b.**

In alternative embodiments, said compartment for temperature conditioning of said fluid is shared between two or more treatment chambers **30a, 30b, 300a, 300b.**

According to the invention, said at least one thermally insulated treatment chamber **30a, 30b, 300a, 300b** further comprises one or more first biocompatible temperature sensors **32a, 32b, 32c, 32d, 32e, 320a, 320b** for each providing a first sensor signal **33a, 33b, 33c, 33d, 33e, 330a, 330b** indicative of the temperature in a corresponding sample of said one or more of said at least one biological sample **20a, 20b, 20c, 20d, 200a, 200b,** wherein each of said biocompatible temperature sensors **32a, 32b, 32c, 32d, 32e, 320a, 320b** is insertable in said corresponding sample.

As used herein, the term "biocompatibility" refers to the ability of a material to perform its desired function, without eliciting any undesirable local or systemic effects in the recipient. In this context, the biocompatible temperature sensor will not have any undesired, such as toxic effects, on the development and activity of the biological sample.

Typically, said one or more first biocompatible sensor **32a, 32b, 32c, 32d, 32e, 320a, 320b** is in contact with, inserted in, or submerged in, a corresponding biological sample **20a, 20b, 20c, 20d, 200a, 200b.** As a consequence, the sample temperature can be measured directly and said first sensor signal **33a, 33b, 33c, 33d, 33e, 330a, 330b** is a direct measurement of the sample temperature.

In embodiments according to the invention, said at least one biological sample **20a, 20b, 20c, 20d, 200a, 200b** contains, or is in contact with, one, two, three or more of said first biocompatible sensors **32a, 32b, 32c, 32d, 32e, 320a, 320b.**

In preferred embodiments, said at least one biological sample **20a, 20b, 20c, 20d, 200a, 200b** contains, or is in contact with, one single biocompatible sensor.

Typically, said one or more first biocompatible temperature sensor **32a, 32b, 32c, 32d, 32e, 320a, 320b** has an accuracy of 0.1°C, preferably 0.05°C, more preferably 0.01°C.

According to the invention, said at least one thermally insulated treatment chamber **30a, 30b, 300a, 300b** further comprises one or more second temperature sensors **34a, 34b, 34c, 340a, 340b** for each providing a second sensor signal **35a, 35b, 35c, 350a, 350b** indicative of the air temperature in said thermally insulated treatment chamber **30a, 30b, 300a, 300b.**

Preferably, said one or more second temperature sensor **34a, 34b, 34c, 340a, 340b** is located inside a corresponding treatment chamber **30a, 30b, 300a, 300b** in close vicinity to the biological sample. In embodiments, said at least one second temperature sensor **34a, 34b, 34c, 340a, 340b** is mounted on or attached to an interior wall of the treatment chamber **30a, 30b, 300a, 300b.**

In embodiments according to the invention, said at least one thermally insulated treatment chamber **30a, 30b, 300a, 300b** contains one, two, three or more of said one or more second temperature sensor **34a, 34b, 34c, 340a, 340b.** In preferred embodiments, said at least one thermally insulated treatment chamber **30a, 30b, 300a, 300b** contains one single second temperature sensor **34a, 34b, 34c, 340a, 340b.** Typically, said one or more second temperature sensor **34a, 34b, 34c, 340a, 340b** has an accuracy of 0.1°C, preferably 0.05°C, more preferably 0.01°C.

According to the invention, the system **10,100,** and methods as described herein, further comprise at least one controller **40a, 40b, 400** configured for steering thermal means **31a, 31b, 310a, 310b** of a corresponding treatment chamber of said at least one treatment chamber **30a, 30b, 300a, 300b** by taking into account at least said one or more first sensor signal **33a, 33b, 33c, 33d, 33e, 330a, 330b** and said one or more second sensor signal **35a, 35b, 35c, 350a, 350b** of said corresponding treatment chamber.

The controller **40a, 40b, 400** is thus configured to receive at least one or more first sensor signal **33a, 33b, 33c, 33d, 33e, 330a, 330b** and at least one or more second sensor signal **35a, 35b, 35c, 350a, 350b** of a treatment chamber **30a, 30b, 300a, 300b** for steering the thermal means of said treatment chamber. Hence, the controller **40a, 40b, 400** is typically configured for deriving from said first **33a, 33b, 33c, 33d, 33e, 330a, 330b** and second **35a, 35b, 35c, 350a, 350b** sensor signals a command for steering, via said thermal means **31a, 31b, 310a, 310b,** the heating and cooling of the interior of a treatment chamber **30a, 30b, 300a, 300b,** thereby affecting the temperature of the biological sample, such that the sample temperature is or remains acceptably close to a target temperature as typically imposed by a thermal treatment protocol. By taking the first sensor signal **33a, 33b, 33c, 33d, 33e, 330a, 330b** into account, said controller **40a, 40b, 400** will make use of a closed loop mechanism using feedback for defining adequate instructions to the thermal means **31a, 31b, 310a, 310b.**

In embodiments, said controller **40a, 40b, 400** comprises or makes use of a PID controller or controlling mechanism.

In embodiments, the temperature of the sample can be kept within 0.1°C deviation, even within 0.05°C deviation and even within 0.01°C deviation, with respect to a sample target temperature by taking into account said first **33a, 33b, 33c, 33d, 33e, 330a, 330b** and second **35a, 35b, 35c, 350a, 350b** sensor signals.

In embodiments according to the invention, said at least one controller **40a, 40b, 400** relates to one single controller for steering the thermal means **31a, 31b, 310a, 310b** of each of said at least one thermally insulated treatment chamber **30a, 30b, 300a, 300b** by taking into account the corresponding said one or more first sensor signal **33a, 33b, 33c, 33d, 33e, 330a, 330b** and the corresponding said one or more second sensor signal **35a, 35b, 35c, 350a, 350b.**

In embodiments according to the invention, the system **10,100,** and methods as described herein, further comprises at least one sensor (not shown on the figures) to monitor one or more of relative humidity, oxygen and carbon dioxide levels inside at least one treatment chamber **30a, 30b, 300a, 300b.** Preferably, the system **10,100,** and methods as described herein further comprises means (not shown on the figures) to regulate one or more of humidity, oxygen and carbon dioxide levels inside at least one treatment chamber **30a, 30b, 300a, 300b.**

In preferred embodiments, said at least one controller **40a, 40b, 400** is configured for receiving an input signal of said sensor for monitoring one or more of relative humidity, oxygen and carbon dioxide levels, and for steering said means to regulate one or more of humidity, oxygen and carbon dioxide levels on the basis of said input signal.

In embodiments according to the invention, at least one treatment chamber **30a, 30b, 300a, 300b** is further provided with at least one of the following:
- a weighing scale for continuously evaluating the weight of said at least one biological sample **20a, 20b, 20c, 20d, 200a, 200b;**
- at least one camera for providing visual inspection of said one or more of said at least one biological sample **20a, 20b, 20c, 20d, 200a, 200b,** and preferably a light source for illuminating said at least one biological sample **20a, 20b, 20c, 20d, 200a, 200b;**
- an optical detection system with fluorescence for inspection and continuously monitoring of said one or more of said at least one biological sample **20a, 20b, 20c, 20d, 200a, 200b;**
- an accelerometer, for evaluating activities such as movements by an egg embryo.

It has been found that such features, individually or in combination, allow to evaluate the effect of a thermal treatment on a biological sample.

According to another aspect of the present invention, there is provided a use of the system **10,100** as described herein for defining, determining or selecting a thermal treatment for treating a biological sample of tumour cells.

It has been found that the observed impact of a specific thermal treatment on a cell culture of tumour cells of an individual patient provides at least partly an indication of the effectiveness of such specific thermal treatment on the body of said individual patient. Such impact or effect can be measured or expressed by optical detection systems, weighing scale, accelerometer, as described herein.

According to another aspect of the present invention, there is provided a method for controlling the temperature of at least one biological sample **20a, 20b, 20c, 20d, 200a, 200b** in a thermally insulated treatment chamber **30a, 30b, 300a, 300b,** the method comprising the following steps:
- placing one or more of said at least one biological sample **20a, 20b, 20c, 20d, 200a, 200b** in said thermally insulated treatment chamber **30a, 30b, 300a, 300b;**
- inserting one or more first biocompatible temperature sensors **32a, 32b, 32c, 32d, 32e, 320a, 320b** in said one or more of said at least one biological sample **20a, 20b, 20c, 20d, 200a, 200b,** each of said one or more first biocompatible temperature sensors **32a, 32b, 32c, 32d, 32e, 320a, 320b** providing a first sensor signal **33a, 33b, 33c, 33d, 33e, 330a, 330b** indicative of the temperature in a corresponding sample of said one or more of said at least one biological sample **20a, 20b, 20c, 20d, 200a, 200b;** and
- steering thermal means **31a, 31b, 310a, 310b** of said thermally insulated treatment chamber **30a, 30b, 300a, 300b** for altering the temperature of said one or more of said at least one biological sample **20a, 20b, 20c, 20d, 200a, 200b** by use of a controller **40a, 40b, 400** by taking into account at least said first sensor signal **33a, 33b, 33c, 33d, 33e, 330a, 330b** and a second sensor signal **35a, 35b, 35c, 350a, 350b** of said corresponding treatment chamber, provided by one or more second temperature sensors **34a, 34b, 34c, 340a, 340b,** each providing a second sensor signal **35a, 35b, 35c, 350a, 350b** indicative of the air temperature in said thermally insulated treatment chamber **30a, 30b, 300a, 300b.**

In embodiments of the method, said method further comprises the following step:
- evaluating and/or comparing an effect or reaction of said at least one biological sample **20a, 20b, 20c, 20d, 200a, 200b** to said temperature alterations, and
- selecting a thermal treatment for treating said biological sample, on the basis of said effect to said temperature alterations.

According to another aspect of the present invention, there is provided a method for defining and/or selecting a thermal treatment for treating a culture of tumour cells, the method comprising the following steps:
- placing one or more of at least one biological sample **20a, 20b, 20c, 20d, 200a, 200b** of said culture of tumour cells in a thermally insulated treatment chamber **30a, 30b, 300a, 300b;**
- inserting one or more first biocompatible temperature sensors **32a, 32b, 32c, 32d, 32e, 320a, 320b** in said one or more of said at least one biological sample **20a, 20b, 20c, 20d, 200a, 200b,** each of said one or more first biocompatible temperature sensors **32a, 32b, 32c, 32d, 32e, 320a, 320b** providing a first sensor signal **33a, 33b, 33c, 33d, 33e, 330a, 330b** indicative of the temperature in a corresponding sample of said one or more of said at least one biological sample **20a, 20b, 20c, 20d, 200a, 200b;**
- steering thermal means **31a, 31b, 310a, 310b** of said thermally insulated treatment chamber **30a, 30b, 300a, 300b** for altering the temperature of said one or more of said at least one biological sample **20a, 20b, 20c, 20d, 200a, 200b** by use of a controller **40a, 40b, 400** by taking into account at least said first sensor signal **33a, 33b, 33c, 33d, 33e, 330a, 330b** and a second sensor signal **35a, 35b, 35c, 350a, 350b** of said corresponding treatment chamber, provided by one or more second temperature sensors **34a, 34b, 34c, 340a, 340b,** each providing a second sensor signal **35a, 35b, 35c, 350a, 350b** indicative of the air temperature in said thermally insulated treatment chamber **30a, 30b, 300a, 300b,**
- evaluating and/or comparing an effect or reaction of said at least one biological sample **20a, 20b, 20c, 20d, 200a, 200b** to said temperature alterations, and
- selecting a thermal treatment for treating said culture of tumour cells, on the basis of said effect to said temperature alterations.

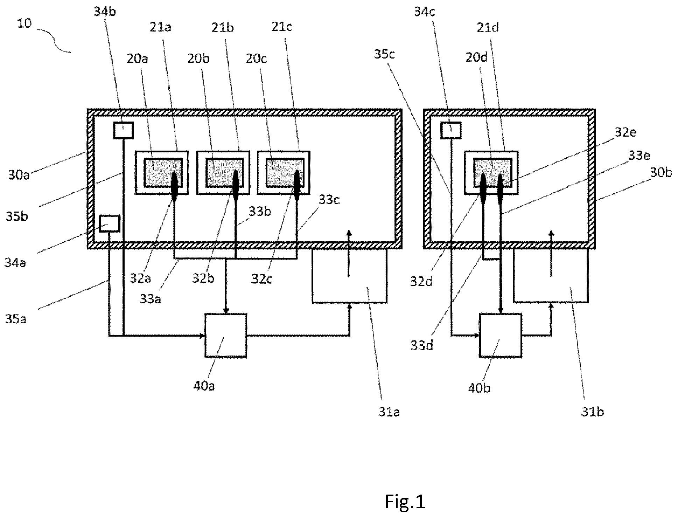

Referring now to the figures, Figure 1 schematically illustrates a system 10 for controlling the temperature of at least one biological sample in at least one thermally insulated chamber according to an embodiment of the present invention. More in particular, Figure 1 illustrates a system 10 with two treatment chambers **30a, 30b,** each chamber being assigned to a controller **40a, 40b** respectively. Treatment chamber **30a** has three biological samples **20a, 20b, 20c,** wherein each of said samples is located in its individual recipient **21a, 21b, 21c** respectively. A first biocompatible temperature sensor **32a, 32b, 32c** respectively, is inserted in each individual sample **20a, 20b, 20c,** and provide first sensor signals **33a,33b,33c** respectively. Chamber **30a** is further provided with two second temperature sensors **34a, 34b,** providing second sensor signals **35a,35b** respectively. Chamber **30a** further comprises a thermal means **31a.** Controller **40a** is configured to receive signals **33a,33b,33c,35a, 35b** and to steer thermal means **31a** for altering the interior temperature of chamber **30a.**

Treatment chamber **30b** has one biological sample **20d,** located in its individual recipient **21d.** First biocompatible temperature sensors **32d, 32e,** are inserted in said sample **20d** and provide first sensor signals **33d,33e** respectively. Chamber **30b** is further provided with a second temperature sensor **34c,** providing a second sensor signal **35c** respectively. Chamber **30b** further comprises a thermal means **31b.** Controller **40a** is configured to receive signals **33d,33e,35c** and to steer thermal means **31b** for altering the interior temperature of chamber **30b.**

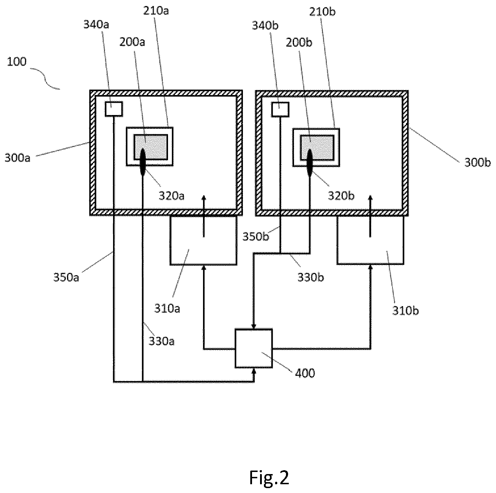

Referring to Figure 2, a system 100 for controlling the temperature of at least one biological sample in at least one thermally insulated chamber according to an embodiment of the present invention is illustrated. More in particular, Figure 2 illustrates a system 100 with two treatment chambers **300a, 300b,** wherein both chambers are assigned to a single controller 400. Treatment chamber **300a** has one biological sample **200a,** located in its individual recipient **210a.** A first biocompatible temperature sensor **320a** is inserted in said sample **200a** and provides a first sensor signal **330a.** Chamber **300a** is further provided with a second temperature sensor **340a,** providing a second sensor signal **350a.** Chamber **300a** further comprises a thermal means **310a.** Controller **400** is configured to receive signals **330a,350a** and to steer thermal means **310a** for altering the interior temperature of chamber **300a.**

Treatment chamber **300b** has one biological sample **200b,** located in its individual recipient **210b.** A first biocompatible temperature sensor **320b** is inserted in said sample **200b** and provides a first sensor signal **330b.** Chamber **300b** is further provided with a second temperature sensor **340b,** providing a second sensor signal **350b.** Chamber **300b** further comprises a thermal means **310b.** Controller **400** is furthermore configured to receive signals **330b,350b** and to steer thermal means **310b** for altering the interior temperature of chamber **300b.**

While the invention has been described hereinabove with reference to specific embodiments, this is done to illustrate and not to limit the invention, the scope of which is defined by the accompanying claims. The skilled person will readily appreciate that different combinations of features than those described herein are possible without departing from the scope of the claimed invention.

## Claims

1. A system (10,100) for controlling the temperature of at least one biological sample (20a, 20b, 20c, 20d, 200a, 200b);
wherein said system (10,100) comprises:
- At least one thermally insulated treatment chamber (30a, 30b, 300a, 300b) suitable for each containing one or more of said at least one biological sample (20a, 20b, 20c, 20d, 200a, 200b), each in its individual recipient (21a, 21b, 21c, 21d, 210a, 210b);
wherein each one of said at least one thermally insulated treatment chamber (30a, 30b, 300a, 300b) further comprises:
o a thermal means (31a, 31b, 310a, 310b) for altering the temperature of said one or more of said at least one biological sample (20a, 20b, 20c, 20d, 200a, 200b), when placed inside said thermally insulated treatment chamber (30a, 30b, 300a, 300b);
o one or more first biocompatible temperature sensors (32a, 32b, 32c, 32d, 32e, 320a, 320b) for each providing a first sensor signal (33a, 33b, 33c, 33d, 33e, 330a, 330b) indicative of the temperature in a corresponding sample of said one or more of said at least one biological sample (20a, 20b, 20c, 20d, 200a, 200b), wherein each of said biocompatible temperature sensors (32a, 32b, 32c, 32d, 32e, 320a, 320b) is insertable in said corresponding sample;
o one or more second temperature sensors (34a, 34b, 34c, 340a, 340b) for each providing a second sensor signal (35a, 35b, 35c, 350a, 350b) indicative of the air temperature in said thermally insulated treatment chamber (30a, 30b, 300a, 300b);
- at least one controller (40a, 40b, 400) configured for steering thermal means (31a, 31b, 310a, 310b) of a corresponding treatment chamber of said at least one treatment chamber (30a, 30b, 300a, 300b) by taking into account at least said one or more first sensor signal (33a, 33b, 33c, 33d, 33e, 330a, 330b) and said one or more second sensor signal (35a, 35b, 35c, 350a, 350b) of said corresponding treatment chamber.

2. The system (10, 100) according to claim 1, wherein said at least one controller (40a, 40b, 400) relates to one single controller for steering the thermal means (31a, 31b, 310a, 310b) of each of said at least one thermally insulated treatment chamber (30a, 30b, 300a, 300b) by taking into account the corresponding said one or more first sensor signal (33a, 33b, 33c, 33d, 33e, 330a, 330b) and said one or more second sensor signal (35a, 35b, 35c, 350a, 350b).

3. The system (10, 100) according to claim 1 or claim 2, wherein each of said at least one thermally insulated treatment chamber (30a, 30b, 300a, 300b) contains one biological sample (20a, 20b, 20c, 20d, 200a, 200b).

4. The system (10, 100) according to any one of claims 1-3, wherein said thermal means (31a, 31b, 310a, 310b) comprise at least one of the following:
- a device for heating and/or cooling fluid and related conducts for circulating said fluid through a corresponding treatment chamber (30a, 30b, 300a, 300b);
- a resistive heating element for heating the air in a corresponding thermally insulated treatment chamber (30a, 30b, 300a, 300b);
- a cooling element for cooling the air in a corresponding thermally insulated treatment chamber (30a, 30b, 300a, 300b);
- a radiation heating element.

5. The system (10, 100) according to any one of claims 1-4, wherein said thermal means (31a, 31b, 310a, 310b) comprise a device for heating and/or cooling fluid and related conducts for circulating said fluid through a corresponding treatment chamber (30a, 30b, 300a, 300b), and a compartment for temperature conditioning of said fluid.

6. The system (10, 100) according to any one of claims 1-5, wherein each one of said at least one thermally insulated treatment chamber (30a, 30b, 300a, 300b) further comprises at least one sensor to monitor one or more of relative humidity, oxygen and carbon dioxide levels inside said treatment chamber (30a, 30b, 300a, 300b).

7. The system (10, 100) according to any one of claims 1-6, wherein each one of said at least one thermally insulated treatment chamber (30a, 30b, 300a, 300b) comprises means to regulate one or more of humidity, oxygen and carbon dioxide levels inside said treatment chamber (30a, 30b, 300a, 300b).

8. The system (10, 100) according to any one of claims 1-7, wherein at least one of said at least one treatment chamber (30a, 30b, 300a, 300b) is further provided with at least one weighing scale for continuously evaluating the weight of said at least one biological sample (20a, 20b, 20c, 20d, 200a, 200b).

9. The system (10, 100) according to any one of claims 1-8, wherein at least one of said at least one thermally insulated treatment chamber (30a, 30b, 300a, 300b) is further provided with at least one camera for providing visual inspection of said one or more of said at least one biological sample (20a, 20b, 20c, 20d, 200a, 200b).

10. The system (10, 100) according to any one of claims 1-9, wherein at least one of said at least one thermally insulated treatment chamber (30a, 30b, 300a, 300b) is further provided with an optical detection system with fluorescence for inspection and continuously monitoring of said one or more of said at least one biological sample (20a, 20b, 20c, 20d, 200a, 200b).

11. The system (10, 100) according to any one of claims 1-10, wherein at least one of said at least one thermally insulated treatment chamber (30a, 30b, 300a, 300b) is further provided with an accelerometer.

12. The system (10, 100) according to any one of claims 1-11, wherein said at least one biological sample (20a, 20b, 20c, 20d, 200a, 200b) refers to one of the following: a cell culture, an egg, preferably a fertilized egg, and wherein said at least one biological sample (20a, 20b, 20c, 20d, 200a, 200b) contains tumour cells.

13. The system (10, 100) according to any one of claims 1-12, wherein said at least one controller (40) comprises a PID controller.

14. Method for controlling the temperature of at least one biological sample (20a, 20b, 20c, 20d, 200a, 200b) in a thermally insulated treatment chamber (30a, 30b, 300a, 300b), the method comprising the following steps:
- placing one or more of said at least one biological sample (20a, 20b, 20c, 20d, 200a, 200b) in said thermally insulated treatment chamber (30a, 30b, 300a, 300b);
- inserting one or more first biocompatible temperature sensors (32a, 32b, 32c, 32d, 32e, 320a, 320b) in said one or more of said at least one biological sample (20a, 20b, 20c, 20d, 200a, 200b), each of said one or more first biocompatible temperature sensors (32a, 32b, 32c, 32d, 32e, 320a, 320b) providing a first sensor signal (33a, 33b, 33c, 33d, 33e, 330a, 330b) indicative of the temperature in a corresponding sample of said one or more of said at least one biological sample (20a, 20b, 20c, 20d, 200a, 200b);
- steering thermal means (31a, 31b, 310a, 310b) of said thermally insulated treatment chamber (30a, 30b, 300a, 300b) for altering the temperature of said one or more of said at least one biological sample (20a, 20b, 20c, 20d, 200a, 200b) by use of a controller (40a, 40b, 400) by taking into account at least said first sensor signal (33a, 33b, 33c, 33d, 33e, 330a, 330b) and a second sensor signal (35a, 35b, 35c, 350a, 350b) of said corresponding treatment chamber, provided by one or more second temperature sensors (34a, 34b, 34c, 340a, 340b), each providing a second sensor signal (35a, 35b, 35c, 350a, 350b) indicative of the air temperature in said thermally insulated treatment chamber (30a, 30b, 300a, 300b).

15. The method according to claim 14, wherein said method further comprises the following steps:
- evaluating and/or comparing an effect or reaction of said at least one biological sample (20a, 20b, 20c, 20d, 200a, 200b) to said temperature alterations, and
- selecting a thermal treatment for treating said biological sample, on the basis of said effect to said temperature alterations.
